# EUROPEAN PATENT APPLICATION

(11) **EP 4 389 094 A1**
(43) Date of publication of application: **26.06.2024**
(21) Application number: 22214629.2
(22) Date of filing: 19.12.2022
(51) Int. Cl.: A61F 13/494, A61F 13/505, A61F 13/514

(54) **ABSORBENT CHASSIS WITH ELASTICIZED OUTER LEG BARRIERS**

(71) Applicant: Ontex BV, 9255 Buggenhout (BE); Ontex Group NV, 9320 Erembodegem (BE)
(72) Inventor: DEL CARMEN SANCHEZ FERNANDEZ, Lucia, Puebla, 72440 (MX); CANALES ESPINOSA DE LOS MONTEROS, Carlos, Puebla, 72813 (MX); VAZQUEZ ARANA, Mauricio, Duluth, Georgia, 30097 (US)
(74) Representative: Larangé, Françoise

(57) **Abstract**

The present disclosure relates to a disposable absorbent chassis (10) having a liquid-pervious topsheet (1), a backsheet (3), and a liquid absorbent core (2) disposed between the liquid-pervious topsheet and the backsheet. The chassis has a pair of longitudinal elasticized standing leg gathers (4, 5) and a pair of elasticized outer leg barriers (30a, 30b) disposed on either side of the longitudinal central axis. Each of the elasticized outer leg barriers (30a, 30b) comprises a substrate folded inward toward the longitudinal central axis, a distal folded edge (31) and a substrate edge (303b, 304b) that is transversely inward from the distal folded edge, such that a folded portion extends between the distal folded edge (31) and the substrate edge (303b, 304b). An elastic member (8a) is disposed within the folded portion at the distal folded edge of the elasticized outer leg barriers (30a, 30b), and each of the elasticized outer leg barriers (30a, 30b) is bonded to one of the lateral flaps (50a, 50b) of the longitudinal elasticized standing leg gathers (4, 5).

## Description

### TECHNICAL FIELD

The present invention relates to a chassis to be used in absorbent disposable articles such as diapers and pants for babies or adults having elasticized outer leg barriers.

### BACKGROUND

Disposable articles like diapers and pants comprising elasticized outer leg barriers are known in the art. A common configuration for the outer leg barriers includes the placement of elastic elements between the topsheet and the backsheet of the disposable article in the crotch portion at the longitudinal side edges of the article.

U.S. Pat. No. 8,795,250 B2 (First Quality) discloses an absorbent article comprising a chassis and an outer leg cuff that is disposed outward of each of the longitudinal side edges of the chassis having elastics forming a gather along at least a portion thereof. One or more inner leg cuffs are disposed on the inside surface of the chassis along each of the longitudinal side edges thereof having elastics forming a gather along at least a portion of the crotch portion. One or more fastening components are disposed at the outer longitudinal side of the outer leg cuffs to fasten the absorbent article around the waist of the wearer. US Pre-Grant Publication No. 20200000648 (Kimberly Clark) discloses an absorbent article that includes leg elastics that form the outermost edges of the product. The leg elastics can include an increased number of elastomeric strands and can extend outboard of the chassis.

U.S. Pat. No. 10,543,131 (P&G) discloses an absorbent article that may include a chassis and a leg gasketing system that may include an inner cuff and an outer cuff; the inner cuff may include an inner cuff folded edge and an inner cuff material edge and the outer cuff may include an outer cuff folded edge and an outer cuff material edge such that the web of material is folded laterally inward to form the outer cuff folded edge and folded laterally outward to form the inner cuff folded edge.

U.S. Pat. No. 9,301,889 (P&G) discloses a disposable absorbent article having an elasticized outer leg cuff that is disposed adjacent to a longitudinal side edge of the absorbent article. The elasticized outer leg cuff has a base and a gasket cuff supported by the base at a joint of the base to the gasket cuff. The gasket cuff is provided with elasticity and has a top gasket cuff surface, an inner cuff extending laterally inwardly from the joint and an outer cuff extending laterally outwardly from the joint. Before use of the absorbent article, the elasticized outer leg cuff is folded laterally inwardly onto the inner surface of the absorbent article such that the top gasket cuff surface faces upwardly.

### SUMMARY

In one aspect, the present disclosure relates to a disposable absorbent chassis having a longitudinal direction along a longitudinal central axis, a transverse direction along a transverse central axis, a user-facing side and a garment-facing side, a front waist portion, a rear waist portion and a crotch portion disposed longitudinally between the front waist portion and the rear waist portion; a first longitudinal side edge and a second longitudinal side edge. The disposable absorbent chassis comprises a liquid-pervious topsheet, a backsheet, a liquid absorbent core disposed between the liquid-pervious topsheet and the backsheet, wherein the backsheet comprises a liquid impervious film layer and an outer cover layer; the liquid impervious film layer having a first longitudinal edge, a second longitudinal edge and a liquid impervious film layer width measured in the transverse direction; the outer cover layer having a first longitudinal edge and a second longitudinal edge and an outer cover width measured in the transverse direction. The disposable absorbent chassis further comprises a pair of longitudinal elasticized standing leg gathers disposed on either side of the longitudinal central axis, each longitudinal elasticized standing leg gather has a lateral flap that extends transversely outward toward one of the longitudinal side edges of the chassis; and a pair of opposed elasticized outer leg barriers disposed on either side of the longitudinal central axis. Each of the elasticized outer leg barriers comprises at least one elastic member, a substrate folded inward toward the longitudinal central axis, a distal folded edge and a substrate edge that is transversely inward from the distal folded edge, such that a folded portion extends between the distal folded edge and the substrate edge. At least one elastic member is disposed within the folded portion at the distal folded edge of the elasticized outer leg barriers, and each of the elasticized outer leg barriers is bonded to one of the lateral flaps of the longitudinal elasticized standing leg gathers.

In further aspects, a disposable absorbent diaper or disposable absorbent pants include the disposable absorbent chassis described herein. In further aspects, an absorbent article comprises a reusable outer cover and the disposable absorbent chassis described herein.

Other objects and advantages of this invention will become apparent hereinafter.

### BRIEF DESCRIPTION OF THE FIGURES

FIG. 1A is a top plan view of the chassis of the present embodiments in a flat configuration, including a first embodiment of the outer leg barriers.
FIG. 1B to 1J are transverse sectional views of the chassis of FIG. 1A, as viewed along A-A, and represent different embodiments of the first and second elasticized outer leg barriers.
FIG. 2 is a top plan view of a disposable diaper in a flat configuration, including the chassis of the present embodiments.
FIG 3 is a top plan view of a disposable pants in a flat configuration, including the chassis of the present embodiments.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure. As used herein, the following terms have the following meanings:
"A", "an", and "the" as used herein refers to both singular and plural unless the context clearly dictates otherwise. By way of example, "a compartment" refers to one or more compartments.

"About" as used herein referring to a measurable value such as a parameter, an amount, a temporal duration, and the like, encompass variations of +/-20% or less, preferably +/-((10))% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants and diaper holders and liners, Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. The absorbent article includes a chassis and may also include other components, such as liquid wicking layers, liquid intake layers, liquid distribution layers, transfer layers, barrier layers, wrapping layers and the like, as well as combinations thereof. Disposable absorbent articles and the components thereof can operate to provide a skin-facing surface and a garment-facing surface.

A "chassis" of a disposable absorbent articles can include a liquid pervious topsheet, a backsheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis comprises a front waist portion, a back waist portion, an intermediate crotch portion which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The terms "back waist portion", "back section" or "back portion" are used herein as synonyms and refer to the area of the absorbent article which is contact with the back of the wearer when the absorbent article is worn.

The term "backsheet" refers to a material forming a liquid impervious cover of the chassis of the absorbent article. The back sheet prevents the exudates contained in the absorbent core from wetting articles such as bedsheets and overgarments which contact the disposable absorbent article. The back sheet may be a composite layer composed of multiple components assembled side-by-side or laminated. The back sheet may be the same or different in different parts of the chassis. At least in the area of the absorbent core, the back sheet comprises a liquid impervious material in the form of a thin plastic film, e.g., a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials, in such laminates, the polymeric film can have the same or a different width than the nonwoven material.

As used herein, the " skin-facing" or "bodyside" or " body-facing" or "user facing" side or surface means that surface of the article or component which is intended to be disposed toward or placed adjacent to the body of the wearer during ordinary use, while the "outward", "outward-facing", "garment-facing" or "garment"" side or surface is on the opposite side, and is intended to be disposed to face away from the wearer's body during ordinary use. Such outward surface may be arranged to face toward or placed adjacent to the wearer's undergarments when the absorbent article is worn.

Further, a chassis can comprise "standing gathers" or "containment flaps" or "barrier cuffs" or "leg cuffs". The standing gathers are generally thought to be particularly well suited for the containment of fecal matter and to prevent the lateral flow of liquid waste until such time as the liquid waste can be absorbed by the absorbent article. Many constructions of standing gather are known. Such standing gather generally comprises a proximal portion intended to be attached to the chassis, and an opposed distal portion which is generally not attached to the chassis along at least a portion of its length. An elastic member is generally located on or adjacent a distal edge to provide elasticity to the standing gather, to assist in maintaining the containment flap in an upright condition and in maintaining a sealing relationship between the distal edge of the standing gather and the body of a wearer during use. The elastic member is generally located between two layers of material so that the elastic does not come into contact with the body of a wearer. The standing gather may be manufactured from any of a wide variety of materials such as woven, non-woven, spunbonded, carded, cast, blown or the like, or combinations or laminations thereof. The materials may include any of a variety of compositions including but not limited to polypropylene, polyester, rayon, nylon, foams, plastic films, formed films, and elastic foams. To improve containment, the material may be hydrophobic, or at least partially liquid impervious. A number of manufacturing techniques may be used to manufacture the containment flaps.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "flat configuration" or "laid-flat state" or "fully stretched state" or "extended state" or flat-out configuration is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven substrate/fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g., superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g., saline with 0.9 wt. % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system and may optionally be employed in other components or portions of the absorbent article. The superabsorbent material may be included in the absorbent layer or other fluid storage layer of the absorbent article of the present invention in an amount up to ((10))0% by weight. Typically, the superabsorbent material, when present, will be included in an amount of from about 30% to about 70% by weight, based on the total weight of the absorbent layer.

"Superabsorbent polymer particles" or "SAPs" refer to water-swellable, water-insoluble organic or inorganic materials capable, under the most favorable conditions, of absorbing at least about ((10)) times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between ((10))0 to 800 µm, preferably between 300 to 600 µm, more preferably between 400 to 500 µm.

The term "topsheet" refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The top sheet can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g., urine or menstrual fluid. The inner coversheet may further be different in different parts of the chassis of an absorbent article. The top sheet fabrics may be composed of a substantially hydrophobic material, and the hydrophobic material may optionally be treated with a surfactant or otherwise processed to impart a desired level of wettability and hydrophilicity.

The term "re-usable outer cover" or "re-usable shell", refers to a component of a disposable absorbent article that is adapted to be worn about the lower torso of a user, and is configured to support and hold a disposable absorbent insert, such as the absorbent chassis described herein. The reusable cover permits removal and replacement of the absorbent insert without substantial destruction of the components of the outer cover that are necessary to the functionality of the outer cover. The reusable outer cover may comprise materials and have a construction that enable it to be re-used and/or may be washed in order to be used more than once.

As used herein, the term "transverse" or "lateral" refers to a line, axis, or direction which lies within the plane of the absorbent article and is generally perpendicular to the longitudinal direction (i.e., is parallel to the x-axis).

Embodiments according to the disclosure will now be described. It is understood that technical features described in one or more embodiments may be combined with one or more other embodiments without departing from the intention of the disclosure and without generalization therefrom.

Generally speaking, aspects of the invention include a chassis to be used in a disposable absorbent article comprising longitudinally-extending elasticized outer leg barriers that have an elastic member disposed at the distal edge of the leg barrier which coincides with the first and second longitudinal side edges of the chassis. The absorbent articles described herein provide improved fit and comfort over conventional absorbent articles, because the outer leg barriers of the embodiments eliminate the undesirable loose edges on the wearer's legs when the absorbent article is worn, and provide a double layer of material between the elastic thread of the outer leg barrier and the skin. This may provide a more premium soft/bulky fit avoiding potential red marks on the skin when the article is in use.

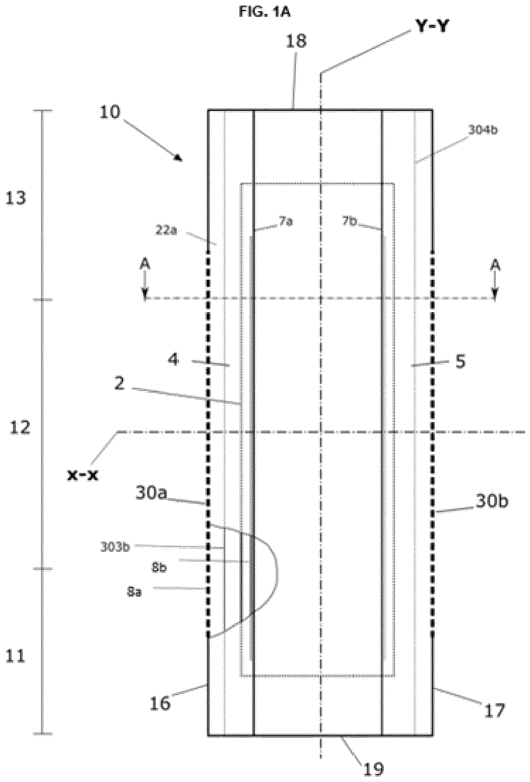

FIG. 1A illustrates a plan view of an exemplary disposable absorbent chassis (10) in an extended state according to an embodiment of the present invention. As can be seen, the chassis (10) comprises a longitudinal central axis Y-Y and a transverse central axis X-X. The longitudinal central axis Y-Y extends along the length of the chassis (10) and the transverse central axis X-X extends along the width of the chassis (10).

The chassis (10) comprises a liquid-pervious topsheet (1) defining at least a portion of the user-facing or skin-facing side of the chassis (10), a backsheet (3) defining at least a portion of the garment-facing side of the chassis (10) and a liquid absorbent core (2) disposed between the liquid-pervious topsheet (1) and the backsheet (3). While the chassis (10) is illustrated in FIG. 1A as having a generally rectangular shape, the chassis (10) could have other shapes or contours, as desired. The chassis (10) comprises a front waist portion (13), a back waist portion (11) and an intermediate crotch portion (12) which interconnects the front and back waist portions. The chassis (10) has a perimeter that is defined, at least in part, by a first longitudinal side edge 16, a second longitudinal side edge 17, a first transverse edge 18, and a second transverse edge 19. The backsheet (3) comprises a liquid impervious film layer (3a) and an outer cover layer (3b). The liquid impervious film layer (3a) comprises a first surface usually facing the wearer's skin, a second surface, usually facing the wearer's garment, a first longitudinal edge (303a), a second longitudinal edge (304a), a first transverse edge (305a) and a second transverse edge (306a) and the outer cover layer (3b) of the backsheet (3) comprises a first surface, usually facing the wearer's skin, a second surface, usually facing the wearer's garment, a first longitudinal edge (303b), a second longitudinal edge (304b), a first transverse edge (305b) and a second transverse edge (306b).

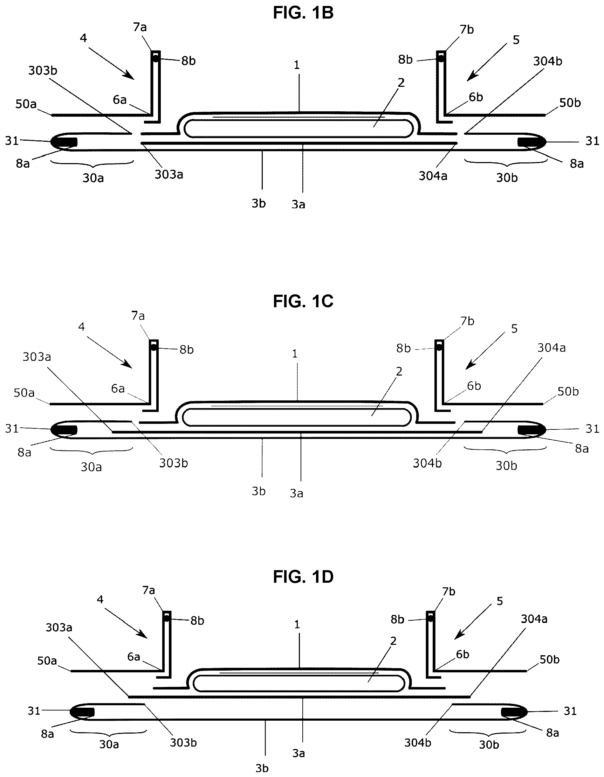

The chassis (10) may comprise first and second longitudinal elasticized standing leg gathers (4, 5) disposed on either side of the longitudinal central axis Y-Y. Each of the first and second longitudinal elasticized standing leg gather (4,5) comprises a proximal portion (6a, 6b) joined to the topsheet, a distal portion (7a,7b) standing away from the topsheet and elasticized by means of at least one elastic member (8b) enclosed within a substrate. Outboard of its attachment to the topsheet, each longitudinal elasticized standing leg gather (4,5) has a lateral flap (50a, 50b) that extends transversely outward toward the longitudinal edges (16, 17) of the chassis (10), respectively. The at least one elastic member (8b) may be attached to the substrate in an stretched condition, joined to the substrate while the substrate is in a gathered state, or joined to the substrate and then elasticized or shrunk, for example with the application of heat, so that elastic retractive forces are imparted to the substrate, or a combination of these methods. The resulting elastic retractive forces will cause the substrate forming the standing leg gather to gather at the distal portion (7a, 7b) and cause the standing leg gather to extend away from the skin-facing surface of the topsheet (e.g., see Fig. 1B) such that the standing leg gather is at least partially extensible in the longitudinal direction.

The chassis (10) includes a pair of opposed elasticized outer leg barriers (30a, 30b) disposed on either side of the longitudinal central axis Y-Y. Each of the outer leg barriers (30a, 30b) has a proximal edge (303b, 304b) proximal to the longitudinal sides of the core, and a distal edge (31) disposed generally at or adjacent the first longitudinal side edge (16), or the second longitudinal side edge (17) of the chassis, respectively. The outer leg barriers (30a, 30b) may extend the entire longitudinal dimension of the longitudinal side edges (16, 17), or only a portion thereof. The elastic members (8a) of the outer leg barriers (30a, 30b) extend at least along the crotch portion and may also extend at least part of the front and/or back waist portions of the chassis (10). The outer leg barriers (30a, 30b) will be described in more detail with reference to the various embodiments shown in FIGS. 1B-1J.

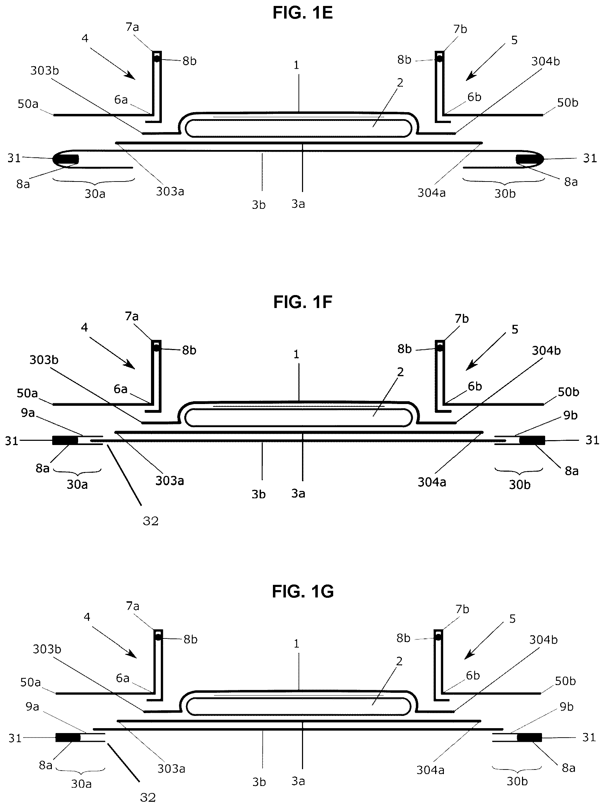

Fig. 1B-1E are cross sectional views of Fig. 1A and show a first set of embodiments in which the backsheet is a laminate of a liquid impervious layer (3a) and an outer cover layer (3b), and the outer leg barriers comprise a portion of the outer cover layer (3b). The liquid impervious layer (3a) is narrower than the outer cover layer (3b), but is wide enough that it covers at least the width of the absorbent core (2). The outer cover layer (3b) extends outward from the longitudinal edges (303a, 304a) of the liquid impervious film (3a), and the elasticized outer leg barriers (30a,30b) include this portion of the outer cover layer (3b). The outer cover layer is folded inward toward the longitudinal central axis, so that the outer leg barriers have a distal folded edge (31) and a substrate edge (303b, 304b) that is transversely inward from the distal folded edge, and a folded portion that extends between the folded edge and the substrate edge forming the outer leg barriers (30a, 30b). As shown in Figs. 1B, 1C and 1D, the outer cover layer (3b) may be folded toward the user-facing side of the chassis so that the folded portion is oriented toward the skin of the user. Alternatively, as shown in Fig. 1E, the outer cover layer (3b) may be folded toward the garment-facing side of the chassis. At least one elastic member (8a) is preferably attached to the outer cover layer (3b) inside the folded portion on or adjacent each of the distal folded edges (31) so that the at least one elastic member (8a) is enclosed in the outer cover layer (3b), forming the elasticized outer leg barriers (30a,30b). The outer leg barriers (30a, 30b) are bonded to the lateral flaps (50a, 50b) of the elasticized longitudinal standing gathers (4, 5). In this way, during use, there are two layers of substrate material (e.g., outer cover 3b, and lateral flaps (50a, 50b) of the standing gather) between the at least one elastic member (8a) and the skin of the wearer. The two substrate layers may help to control, reduce, or avoid red marks on skin caused by the elasticized outer leg barriers, and make the outer leg barriers more comfortable.

Making reference to Fig. 1B, the topsheet (1) and the liquid impervious layer (3a), are coterminous and the substrate edges (303b,304b) do not touch the longitudinal edges of the topsheet and the liquid impervious film layer. In other alternatives shown in Figs. 1C-1E, the liquid impervious film layer (3a) may optionally extend into at least a portion of the outer leg barrier (30a, 30bà. This may, for example, improve the liquid barrier properties in this region if desired. Referring to Fig. 1C, the liquid impervious film layer (3a) may extend at least partially inside the folded portion of the outer cover layer (3b). As shown in FIG. 1D and 1E, the liquid impervious film layer 3a, may extend at least partially between the folded portion of the outer cover layer and the lateral flaps (50a, 50b) of the elasticized longitudinal standing gathers (4, 5).

As explained hereinabove, the elasticized outer leg barriers (30a, 30b) may be integral with the outer cover layer (3b), alternatively, they may be separately joined thereto. For example, in the embodiments shown in Fig. 1F to 1J, the first and second elasticized outer leg barriers (30a, 30b) comprise a wrapper element (9a, 9b) that is a folded substrate joined to one or both layers of the backsheet, and which forms the folded portion of the elasticized outer leg barriers (30a, 30b). The wrapper element has a folded distal edge (31), and a proximal edge (32) in which the wrapper element substrate edges meet. The folded portion of the outer leg barriers spans the distance between the folded distal edge and the proximal substrate edges of the wrapper element. The at least one elastic member (8a) is disposed within the folded portion, at or adjacent the distal folded edge (31), along both the first and second elasticized outer leg barriers (30a, 30b). The wrapper element may be joined to the outer cover layer (3b), and optionally the liquid impervious film layer (3a) in various different configurations explained later. The outer leg barriers (30a, 30b), including the respective wrapper elements (9a, 9b), are bonded to the lateral flaps (50a, 50b) of the elasticized longitudinal standing gathers (4, 5).

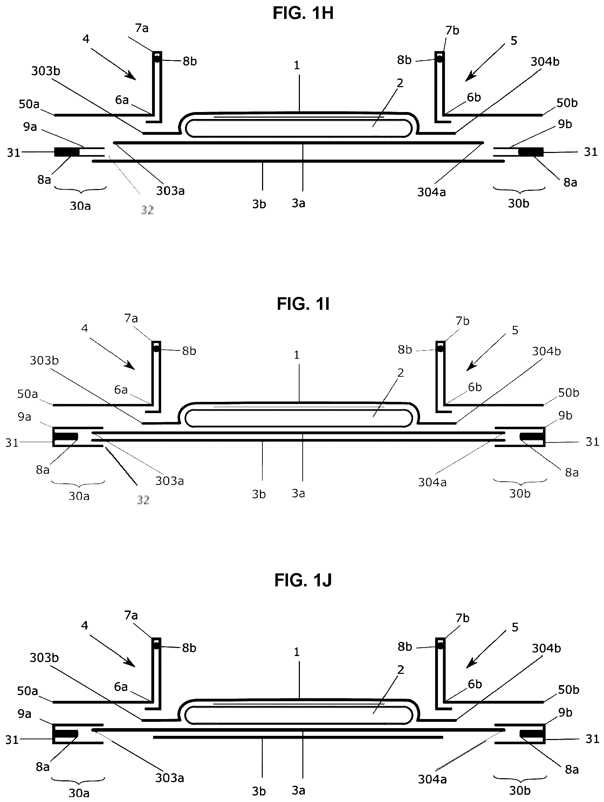

The wrapper elements (9a,9b) may comprise a substrate that is the same as or is different from the outer cover layer 3b, the standing gather (9a,9b), or both, such as a nonwoven or a nonwoven-film laminate. An example of such substrate is the 15 gsm HO Super soft SMS sold by Fitesa Sweden AB under reference SC6KW-30 15NN. The wrapper element (9a,9b) may be joined to the outer cover (3b), the liquid impervious film layer (3a), or both, in any of various configurations. For example, the wrapper element (9a,9b) may be joined to a garment-facing side (301b) of the outer cover (3b) as shown in FIG. 1G, to the user-facing surface (302b) of the outer cover 3b as shown in FIG. 1H, or to both garment-facing and user-facing surfaces of the outer cover (3b) as shown in FIG. 1F. In this latter configuration the longitudinal edges of the outer cover layer extend at least partially into the folded portion such that the folded wrapper elements (9a, 9b) sandwich the outer cover layer (3b) in the folded portion. In other embodiments, the longitudinal edges of the outer cover layer and the longitudinal edges of the liquid impervious film layer may be coterminous and may extend at least partially into the folded portion such that the wrapper elements (9a, 9b) sandwich the outer cover layer (3b) and the liquid impervious film layer (3a) in the folded portion as shown in FIG. 1I. In still other embodiments, as for example shown in FIG. 1J, the outer cover (3b) is narrower than the liquid impervious film layer (3a), and only the liquid impervious film layer (3a) extends at least partially into the folded portion such that the folded wrapper elements (9a, 9b) sandwich the liquid impervious film layer (3a) in the folded portion. The wrapper element (9a,9b) may be joined to the outer cover (3b), the liquid impervious film layer (3a), or both by any suitable means, such as adhesive bonding, mechanical bonding, or a combination thereof. In all described configurations the outer leg barriers (30a, 30b) are joined to the lateral flaps (50a,50b) of the elasticized longitudinal standing gathers.

In cases where wrapper elements (9a, 9b) are used, the backsheet may be formed of only one layer of liquid impervious or hydrophobic material. The backsheet (3) then consists of the liquid impervious film layer (3a); it does not comprise an outer cover layer (3b). The wrapper element, in such case, is joined as described hereinabove to this liquid impervious film layer: on the wearer facing side, on the garment facing side or sandwiching the layer.

While the outer leg barriers (30a, 30b) are illustrated with just one cuff elastic member (8a), one or more additional elastic members may be included in the outer leg barrier. In some embodiments, just one elastic member may be desirable for the disposable article in which the chassis (10) will be included in order to be pretty similar to an undergarment. Each of the at least one elastic members (8a) may be sheets, strands or ribbons of natural rubber, synthetic rubber, thermoplastic elastomeric polymers or any other elasticized material known in the art. Each of the at least one elastic member (8a) may be joined to the outer leg barrier (30a, 30b) using adhesive bonding, mechanical bonding, or a combination thereof so that the outer leg barrier (30a, 30b) is at least partially extensible in the longitudinal direction. Each of the at least one elastic member (8a) may be attached to the leg barrier (30a, 30b) in an stretched condition, while the leg barrier is in a gathered state, or joined to the leg barrier and then elasticized or shrunk, for example with the application of heat, so that elastic retractive forces are imparted to the leg barriers.

In some embodiments, the at least one elastic member 8a can include active portions (i.e., portions of the elastic members that are elastically extensible) and inactive portions (i.e., portions of the elastic members that are non-elastic). Portions of the at least one elastic member (8a) can be rendered inactive, for example, by cutting or otherwise "deadening" the elastic member(s) along a desired inactive portion. The at least one elastic member (8a) can include any of a number of active and/or inactive portions having desirable dimension and configuration.

As told, the chassis with the outer leg barriers (30a, 30b) described herein may improve the comfort and the skin wellness of the wearer, in part because there are two substrate layers between the leg elastics and the skin of the user. In some embodiments, the comfort of the outer leg barriers may be further improved by providing soft substrates in one or more of the standing leg gathers (4, 5), the outer cover (3a), the wrapper element (9a, 9b), or a combination thereof. One way to quantify softness, smoothness, and/or stiffness of a substrate is with an Emtec Tissue Softness Analyzer, according to the Emtec Tissue Softness Test Method described herein; the Tactile softness is measured as TS7 and the Texture/Smoothness is measured as TS750. The elasticized outer leg barriers (30a,30b) and/or the elasticized longitudinal standing leg gathers (4,5) may have one or more substrates with a TS7 (softness) value between about 2 dB V² rms to about 8 dB V² rms, about 2 dB V² rms to about 6.5 dB V² rms or about 2 dB V² rms to about 5.5 dB V² rms. The elasticized outer leg barriers (30a,30b) and/or the standing leg gathers (4,5) may have a TS750 (smoothness) value between about 3 dB V² rms to about 20 dB V² rms, about 3 dB V² rms to about 15 dB V² or about 4 dB V² rms to about 13 dB V² tested according to the Emtec test method included below, improving the softness and protecting the skin of the user. Examples of these materials are a commercially available airthrough bonded nonvowven containing 50% polypropylene, 50% polyethylene with a weight between 15 and 25 gsm and having a TS7 value around 7 and a TS750 value around 12 or a commercially available thermobonded nonwoven made 100% of polypropylene, having a weight between 12 and 25 gsm and a TS7 value around 3.5 and a TS750 value around 10.5.

Another aspect of this invention is an absorbent article, such as an absorbent diaper and/or absorbent pants, that includes a chassis (10) as described herein. The chassis (10) could be a separate sub-assembly that is joined with one or more additional components of the absorbent article, it can be formed integrally with one or more components of the absorbent articles, or a combination thereof. It will be understood that these absorbent articles can optionally contain other features and elements such as a fastening system, a pair of front ear panels, a pair of back ear panels, a front belt, a back belt, elastic waist features, acquisition/distribution layers, front and back barriers , a pair of elastic or inelastic panels, front and back belts, or any other elements known in the art.

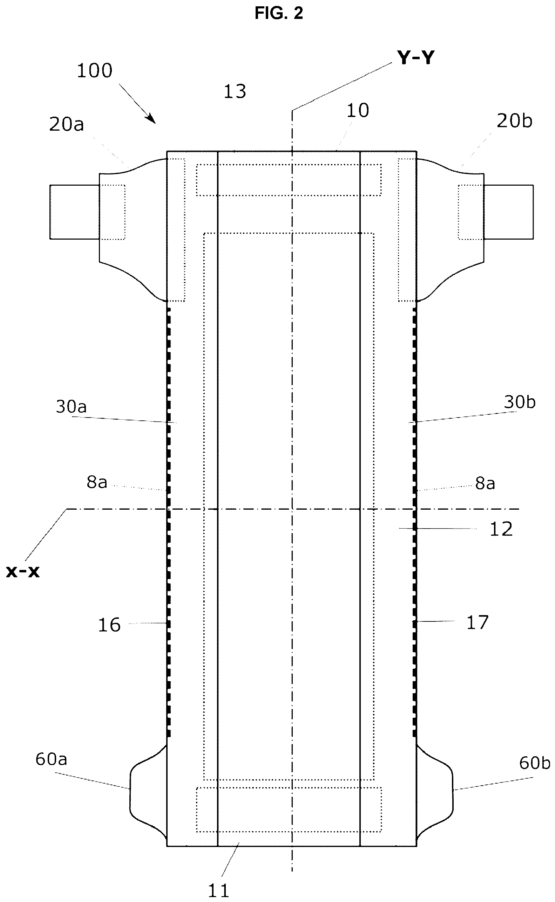

Referring to FIG. 2, in one aspect, a disposable absorbent diaper (100), such as a diaper intended for use by an adult, a baby, or a child, includes the chassis (10) of the present invention. In this aspect, the chassis (10) is formed integrally with the diaper (100), so that the outer cover of the chassis 10 forms the outer cover of the diaper 100. The chassis may further include an acquisition/distribution layer between the core and the topsheet and/or between the core and the backsheet; the diaper 100 may include a pair of opposed back ear panels (20a, 20b) joined to the longitudinal side edges (16, 17) of the chassis (10) at the back waist portion 13, and extending outward. The disposable absorbent article (100) may include a pair of opposed frontal ears (60a,60b) which are joined to longitudinal side edges (16,17) the chassis (10) in the front waist portion (11) and extend outward therefrom. The front ear panels (60a, 60b), back ear panels (20a, 20b) or both may be elastic, inelastic, or a combination thereof. The ear panels, may include one or more elastic or inelastic materials such as a nonwoven, a film, a foam, a filament or strand, or a combination thereof, such as a laminate of two or more materials. For example, the ear panels may include a laminate of an elastic film and a nonwoven material that is extensible in the transverse direction. The ear panels may be attached to the chassis by adhesive, mechanical, thermal bonding, or a combination thereof. One or more fastening members may be joined, directly or indirectly, to the front ear panels, back opposed ear panels, or both. For example, mechanical fasteners may be provided at or near the distal edges of the back ear panels 20a, 20b, that are configured to releasably engage with a corresponding material on front waist portion 11, to enable the diaper 100 to be fastened about the waist of a user. Exemplary fastening means include tape tab fasteners, snaps, pins, belts, hooks, buckles, "hook/mushroom"- and-loop fasteners (e.g. VELCRO^{®}-type fasteners) and the like. The diaper (100) may also include other features such as elastic waist features or barriers in the back portion (13), front portion (11), or both.

The outer leg barriers (30a, 30b) of the chassis (10) extend longitudinally on the diaper 100. The at least one elastic member (8a) of the elasticized outer leg barriers (30a, 30b) , extend over the crotch portion of the chassis (10) and at least into part of the front waist portion (11) and back waist portion (13). For example, referring to FIG. 2, the elastic members (8a) are shown extending between the back ear panels (20a, 20b) and optional frontal ears (60a, 60b), passing through the crotch portion (12). When the diaper is fastened about the waist of the user, the elasticized outer leg barriers (30a, 30b) can form a gasket about the legs of the user to control or prevent liquid leakage.

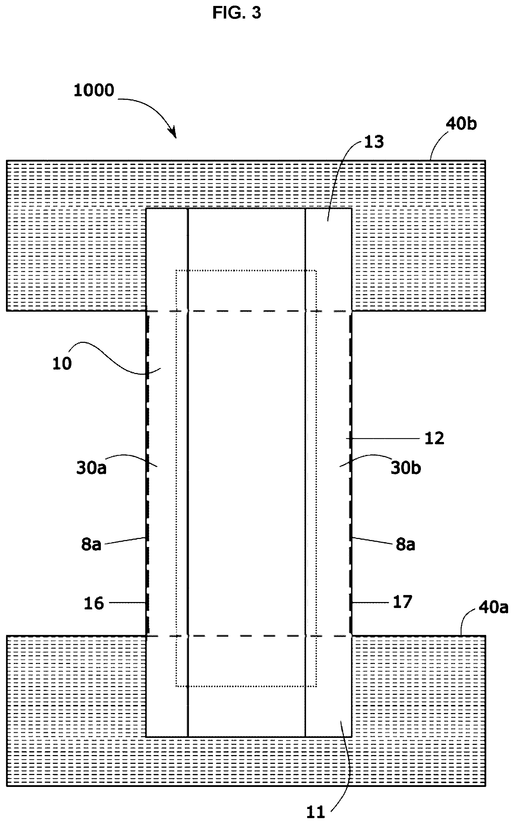

In another aspect, a disposable absorbent pant, such as an adult pant, a child pant, or a baby pant, may include a chassis (10) as described herein. For example, referring to FIG. 3, disposable pants (1000) may comprise a front elasticized belt (40a) adjacent a front waist portion (11) and a back elasticized belt 40b adjacent a back waist portion (13) of the chassis (10), the elasticized belts are extensible in the transverse direction (e.g., parallel to transverse axis X-X). The front and back elasticized belts may comprise an inner nonwoven layer, an outer nonwoven layer and one or more elastic materials (i.e., elastic film, elastic strand) disposed between the inner and outer nonwoven layers. The inner and outer nonwoven layers may be joined to each other and/or to the elastics using adhesive, thermal, or mechanical bonds, or a combination thereof. The front elasticized belt 40a and a back elasticized belt 40b can be sealed together at lateral side seams to form an underwear-resembling product that can be pulled up over the legs of a user and may be removed either by pulling it down in the opposite direction or by tearing the side seams. It will be understood that a fastener system can be provided as an alternative to the side seam, so that the plant is refastenable. The front and back elasticized belts have proximal transverse edges (405a,405b) and first and second distal transverse edges (406a,406b) at the waist portion of the pants.

The chassis (10) may be joined directly or indirectly with the front and back belts and extending longitudinally therebetween to form an absorbent article. For example, the disposable absorbent pant (1000) may have an outer cover that extends the full length of the pant (1000), and each of the front belt, the back belt, and the chassis (10) are joined to the outer cover. As an alternative, the chassis 10 may be directly joined to the front belt and the back belt, extending therebetween, with the outer cover 3b of the chassis (10) forming a portion of the outer cover of the pant. The elastic members (8a) of the elasticized outer leg barriers (30a, 30b) of the chassis (10), extend preferably along the longitudinal edges (16,17) of the chassis, between the front belt and back belt passing through the crotch portion. The standing gathers, the outer leg barriers, or both, can optionally intersect the front belt, the back belt, or both. During use, the elasticized outer leg barriers (30a, 30b) can form a gasket about the legs of the user to control or prevent lateral liquid leakage.

While the pants are described particularly with reference to pants having a front and back belt, it will be understood that the chassis (10) could be incorporated into a pant that has elasticized side panels. For example, a pair of back elasticized side panels could be joined to the longitudinal side edges (16, 17) of the chassis (10) at the back waist portion, extending laterally outward therefrom. And a pair of frontal elasticized side panels could be joined to longitudinal side edges (16,17) the chassis (10) in the front waist portion, extending laterally outward therefrom. The respective front and back elasticized side panels can be coupled (e.g., with a side seam or corresponding fasteners) to form a closed pant product. In this embodiment, the elastic members (8a) of the elasticized outer leg barriers (30a, 30b) of the chassis (10) extend over the crotch portion (12) and between the front and back elasticized panels.

In yet another aspect, an absorbent article can include a re-usable outer shell that is coupled with the chassis (10) (e.g., as a disposable absorbent "insert"). The absorbent article may be adapted to be worn about the lower torso of a user similar to a diaper or pant, described herein. The re-usable outer shell may be adapted to allow insertion/removal of an absorbent chassis (10), without damaging or destroying any necessary re-usable cover components to provide the substantial like-new functionality of the re-usable cover. The standing leg gathers and outer leg barriers help to contain the bodily exudates on the chassis, and prevent them from soiling the outer cover during use. In this embodiment (not shown), the absorbent insert (10), the re-usable outer shell, or both can include fastening means to help position and removably fasten the absorbent chassis to the re-usable outer shell during use. For example, the chassis, the outer cover, or both may include fastening elements such as adhesive, hook-loop fastening system buttons, snaps, pockets, slings, straps, flaps, or any other suitable fastening systems.

### TEST METHODS

### Emtec Tissue Softness Test Method

Softness (TS7), smoothness (TS750), and stiffness (D) properties of different sheet materials may be analyzed using an Emtec Tissue Softness Analyzer ("Emtec TSA"), available from Emtec Electronic GmbH, Leipzig, Germany, interfaced with a computer running Emtec TSA software. The Emtec TSA uses acoustic waves and has demonstrated to correlate well with hand panel tests for thin materials like tissue or nonwoven. The Emtec Tissue Softness test method may be used for determining softness, smoothness of a sheet material such as the topsheet, backsheet, standing gather and outer leg barriers as described herein.

The Emtec TSA includes a rotor with vertical blades which rotate on the test sample at a defined and calibrated rotational speed (set by manufacturer) and contact force of 100 mN. Contact between the vertical blades and the test sample creates vibrations both in the blades and in the test piece, and the resulting sound is recorded by a microphone within the instrument. The recorded sound file is then analyzed by the Emtec TSA software to determine TS7 and TS750 values. The D value is a measure of sample stiffness and is based on the vertical distance required for the contact force of the blades on test sample to be increased from 100 mN to 600 mN.

All measurements and calibrations shall be performed at standard climatic conditions of 23° C (±1° C) and 50% r.H. (±5%) in general following ISO DIN EN 20187.

The TSA is equipped with a balance for determination of grammage and thickness (gravimetric) of the samples in accordance with ISO 12625-6:2005.

### Sample preparation:

A test sample is prepared by cutting a square or circular portion of substrate/structure of interest from the absorbent article (e.g., outer cover, wrapper element, and/or standing leg gather). It is preferable that freeze spray is not used to remove the portion of the substrate to be analyzed, though it is acceptable to use freeze spray in a distal region to aid in initiating the separation of layers. Test samples are cut to a length and width (diameter in the case of a circular sample) of no less than about 90 mm and no greater than about 120 mm to ensure the sample can be clamped into the TSA instrument properly. (If an absorbent article does not contain a sufficiently large area of the substrate of interest to extract a sample of the size specified above, it is acceptable to sample equivalent material from roll stock.) Test samples are selected to avoid unusually large creases or folds within the testing region. Six substantially similar replicate samples are prepared for testing.

All samples are equilibrated at TAPPI standard temperature and relative humidity conditions (23° C.±2 C.° and 50%±2%) for at least 2 hours prior to conducting the TSA testing, which is also conducted under TAPPI conditions.

### Testing procedure:

The instrument is calibrated according to the Emtec's instructions using the 1-point calibration method with the appropriate reference standards (so-called "ref.2 samples," or equivalent, available from Emtec).

A test sample is mounted in the instrument with the surface of interest facing upward, and the test is performed according to the manufacturer's instructions. The software displays values for TS7 and TS750, when the automated instrument testing routine is complete. TS7 and TS750 are each recorded to the nearest 0.01 dB V² rms. The test sample is then removed from the instrument and discarded. This testing procedure is performed individually on the corresponding surfaces of interest of each of the six of the replicate samples (user-facing surface for standing leg gather samples and garment-facing surface for outer cover and wrapper element material samples).

The value of TS7 and, TS750 are each averaged (arithmetic mean) across the six sample replicates. The average values of TS7 and TS750 are reported to the nearest 0.01 dB V² rms.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

It is assumed that the present disclosure is not restricted to any form of embodiment described above and that some modifications can be made to the presented embodiments of the invention without departing from the scope of the embodiments as set forth in the claims that follow.

## Claims

1. A disposable absorbent chassis (10) having a longitudinal direction along a longitudinal central axis (Y-Y), a transverse direction along a transverse central axis (X-X), a user-facing side and a garment-facing side, a front waist portion (11), a rear waist portion (13) and a crotch portion (12) disposed longitudinally between the front waist portion (11) and the rear waist portion (13); a first longitudinal side edge (16) and a second longitudinal side edge (17),
said disposable absorbent chassis (10) comprising a liquid-pervious topsheet (1), a backsheet (3), and a liquid absorbent core (2) disposed between the liquid-pervious topsheet (1) and the backsheet (3),
wherein the backsheet (3) comprises a liquid impervious film layer (3a) and an outer cover layer (3b), the liquid impervious film layer (3a) having a first longitudinal edge (303a), a second longitudinal edge (304a) and a liquid impervious film layer width measured in the transverse direction, the outer cover layer (3b) having a first longitudinal edge (303b) and a second longitudinal edge (304b) and an outer cover layer width measured in the transverse direction;
said disposable absorbent chassis (10) further comprising a pair of longitudinal elasticized standing leg gathers (4, 5) disposed on either side of the longitudinal central axis (Y-Y), wherein each longitudinal elasticized standing leg gather (4, 5) has a lateral flap (50a, 50b) that extends transversely outward toward one of the longitudinal side edges (16, 17) of the chassis (10); and
a pair of opposed elasticized outer leg barriers (30a, 30b) disposed on either side of the longitudinal central axis Y-Y,
**characterized in that** each of the elasticized outer leg barriers (30a, 30b) comprises at least one elastic member (8a), a substrate folded inward toward the longitudinal central axis, a distal folded edge (31), and a substrate edge (303b, 304b) that is transversely inward from the distal folded edge, such that a folded portion extends between the distal folded edge (31) and the substrate edge (303b, 304b), and
wherein at least one elastic member (8a) is disposed within the folded portion at the distal folded edge of the elasticized outer leg barriers (30a, 30b), wherein each of the elasticized outer leg barriers (30a, 30b) is bonded to one of the lateral flaps (50a, 50b) of the longitudinal elasticized standing leg gathers (4, 5).

2. A disposable absorbent chassis according to claim 1, **characterized in that** the substrate of the elasticized outer leg barriers (30a, 30b) comprises a portion of the outer cover layer (3b) that is folded toward the user-facing side or toward the garment-facing side of the absorbent chassis.

3. A disposable absorbent chassis according to claim 1, **characterized in that** the substrate of the elasticized outer leg barriers (30a, 30b) comprises a wrapper element (9a, 9b) separately joined to the backsheet (3) and forming the folded portion.

4. A disposable absorbent chassis according to claim 3, **characterized in that** the wrapper element is joined to a garment-facing side of the outer cover layer (3b), a user-facing side of the outer cover layer (3b), or both the garment-facing side and the user-facing side of the outer cover layer (3b).

5. A disposable absorbent chassis according to any of the preceding claims, **characterized in that** the elasticized outer leg barriers (30a, 30b) comprise a portion of the liquid impervious film layer (3a).

6. A disposable absorbent chassis according to claim 5, **characterized in that** the liquid impervious film layer (3a) is disposed at least partially between the substrate of the elasticized outer leg barriers (30a, 30b) and the lateral flaps (50a, 50b) of the longitudinal elasticized standing leg gathers (4, 5), or at least partially within the folded portion.

7. A disposable absorbent chassis according to any of the preceding claims, **characterized in that** the liquid impervious film layer width is narrower than the outer cover layer width.

8. A disposable absorbent chassis according to any of claims 3 to 4, **characterized in that** the longitudinal edges of the outer cover layer (3b) and the longitudinal edges of the liquid impervious film layer (3a) are coterminous and are disposed within the folded portion of the wrapper elements (9a, 9b).

9. A disposable absorbent chassis according to any of the preceding claims, **characterized in that** the substrate of the elasticized outer leg barriers (30a, 30b) has one or both of: a TS7 value between 2 dB V² rms and 8 dB V² rms measured according to the Emtec test method; and a TS750 value between 3 dB V² rms and 20 dB V² rms measured according to the Emtec test method.

10. A disposable absorbent chassis according to any of the preceding claims, **characterized in that** the lateral flaps (50a, 50b) of the longitudinal elasticized standing leg gathers (4, 5), has one or both of: a TS7 value between 2 dB V² rms and 8 dB V² rms measured according to the Emtec Tissue Softness test method; and a TS750 value between 3 dB V² rms and 20 dB V² rms measured according to the Emtec Tissue Softness test method.

11. A disposable absorbent chassis according to any of the preceding claims, **characterized in that** each of the elasticized outer leg barriers (30a, 30b) comprises two or more elastic members.

12. A disposable absorbent diaper (100) including a disposable absorbent chassis according to any of the preceding claims.

13. A disposable absorbent diaper (100) according to claim 12, wherein said disposable absorbent diaper includes a pair of opposed back ear panels (20a, 20b) joined to the longitudinal side edges (16, 17) of the chassis (10) at the back waist portion (13) and a pair of opposed frontal ears joined to the longitudinal side edges (16,17) of the chassis (10) in the front portion (11), **characterized in that** the at least one elastic member (8a) of the elasticized outer leg barriers (30a, 30b) extends between the back ear panels (20a, 20b) and the front ear panels (60a, 60b).

14. A disposable absorbent pant (1000) including a disposable absorbent chassis according to any of claims 1 to 11.

15. A disposable absorbent pant (1000) according to claim 14, wherein said disposable absorbent pant includes:
- a front elasticized belt (40a) attached and being adjacent to the front waist portion (11) of the chassis (10)
- a back elasticized belt (40b) attached and being adjacent to the rear waist portion (13) of the chassis (10)
**characterized in that** the at least one elastic member (8a) of the elasticized outer leg barriers (30a, 30b) extends between the front elasticized belt (40a) and the back elasticized belt (40b).

16. An absorbent article comprising a re-usable outer shell and a disposable absorbent chassis according to any of claims 1 to 11.
